# EUROPEAN PATENT APPLICATION

(11) **EP 2 388 033 A1**
(43) Date of publication of application: **23.11.2011**
(21) Application number: 09838428.2
(22) Date of filing: 11.03.2009
(51) Int. Cl.: A61M 5/32

(54) **TUBE ASSEMBLY FOR CONTROLLING LENGTH OF SYRINGE NEEDLE**

(30) Priority: 14.01.2009 KR 20090002991
(71) Applicant: Bang, Si-Yeul, Seoul 135-527 (KR)
(72) Inventor: Bang, Si-Yeul, Seoul 135-527 (KR)
(74) Representative: Dr. Weitzel & Partner
(86) International application number: PCT/KR2009/001200
(87) International publication number: WO 2010/082707

(57) **Abstract**

The present invention relates to a tube assembly for controlling the length of a syringe needle. The tube assembly comprises: a coupling body wherein one end is coupled with a needle assembly having a fixing member coupled to the upper end of a syringe cylinder and a syringe needle coupled to the fixing member; and a control tube formed to enable control of exposure length of the syringe needle by being movably coupled to the coupling body along a longitudinal direction of the syringe needle. The coupling body comprises: a coupling part formed on a lower side thereof to be inserted into and coupled to the fixing member, and a through-hole formed therein in order for the syringe needle to penetrate the through-hole and to protrude out of the front end.

## Description

### TECHNICAL FIELD

The present invention relates to a tube assembly for controlling the length of a syringe needle. More particularly, the present invention relates to a tube assembly for controlling the length of a syringe needle, which can be coupled to a typical syringe provided with a syringe needle and can easily control the length of the syringe needle that is exposed.

### BACKGROUND ART

Diverse methods for getting an injection, such as subcutaneous injection, intracutaneous injection, intramuscular injection (I.M), and intravenous injection (I.V), have been used, and such injection methods require not only different operations but also different kinds of syringes. In operation, a standardized syringe needle that meets with the corresponding purpose should be used.

In accordance with the thickness and the length of the syringe needle, the syringe needle standard is determined, and it is required to use an appropriate standardized syringe needle that meets with the corresponding operation method. In addition to the standard of the syringe needle, the injection depth acts as an important factor during operation. For example, if the injection is not made sufficiently deep, but is made near the skin in the case of the injection lipolysis, skin pigmentation may remain, while if the injection is made too deep, muscle may be destructed to cause a muscular ache. In consideration of this point, an operator must pay careful attention so that an injection can be medicated at an accurate medication point.

Medication using a syringe may be mainly given by a specialist such as a doctor or a nurse, and may be given by a non-specialist such as a patient or a patent's family, for example, in the case of insulin injection or the like. In the latter case, more careful attention must be paid. In any case, a syringe needle having an appropriate length must be used in consideration of a pain that occurs during the injection, an injection region, a subcutaneous fat tissue, and the like, and an accurate injection depth is required.

The length of the syringe needle is important in determining an appropriate injection position, and a relatively long syringe needle may cause a patient to feel insecure.

Further, separate forming of syringe needles having diverse lengths may cause the increase of the manufacturing cost, and separate using of syringe needles having different lengths for respective operations may impose an economical burden.

### DISCLOSURE

### TECHNICAL PROBLEM

Therefore, the present invention has been made to solve the above-mentioned problems occurring in the related art, and one embodiment of the present invention is related to a tube assembly for controlling the length of a syringe needle, which can be easily coupled to a typical syringe and can conveniently control the length of the syringe needle that is exposed.

### TECHNICAL SOLUTION

In accordance with an aspect of the present invention, there is provided a tube assembly for controlling the length of a syringe needle, which includes a coupling body coupled to a needle assembly having a fixing member coupled to an upper end of a syringe cylinder and a syringe needle coupled to the fixing member, the coupling body including a coupling part formed on a lower side thereof to be inserted into and coupled to the fixing member, and a through-hole formed therein in order for the syringe needle to penetrate the through-hole and to protrude out of the front end; and a control tube movably coupled to the coupling body along a longitudinal direction of the syringe needle to be able to control an exposure length of the syringe needle.

### ADVANTAGEOUS EFFECT

As described above, according to an embodiment of the present invention, the following effects can be obtained.

First, the exposure length of the syringe needle that is used in a typical syringe can be easily controlled using the coupling body that is coupled to the fixing member of the needle assembly and the control tube movably coupled to the coupling body.

Second, since the coupling body and the control tube are screw-engaged with each other, the degree of exposure of the syringe needle can be easily controlled, and the control tube is prevented from being pushed backward during injection.

Third, since the coupling body is shaped to correspond to the shape of the front end portion of the fixing member, the coupling body and the fixing member are firmly coupled to each other, and the syringe needle having a typical length can be used to perform an operation that requires a syringe needle having a relatively short length.

Fourth, since the control tube is formed to have a polygonal cross-section, or protrusions are formed on an outer circumference of the control tube, the gripping or the position control of the control tube can be easily performed.

### BRIEF DESCRIPTION OF THE DRAWINGS

The above objects, other features and advantages of the present invention will become more apparent by describing the preferred embodiments thereof with reference to the accompanying drawings, in which:
Fig. 1 is an exploded perspective view illustrating a tube assembly for controlling the length of a syringe needle according to an embodiment of the present invention;
Fig. 2 is a perspective view illustrating a state where the tube assembly for controlling the length of a syringe needle illustrated in FIG. 1 is coupled to a needle assembly;
Figs. 3 and 4 are cross-sectional views illustrating a state where the tube assembly for controlling the length of a syringe needle according to another embodiment of the present invention is coupled to a fixing member of a needle assembly;
Fig. 5 is a perspective view and a cross-sectional view illustrating a control tube according to still another embodiment of the present invention; and
Fig. 6 is a perspective view illustrating a control tube according to still another embodiment of the present invention.

### BEST MODE

Now, preferred embodiments of the present invention will be described in detail with reference to the accompanying drawings.

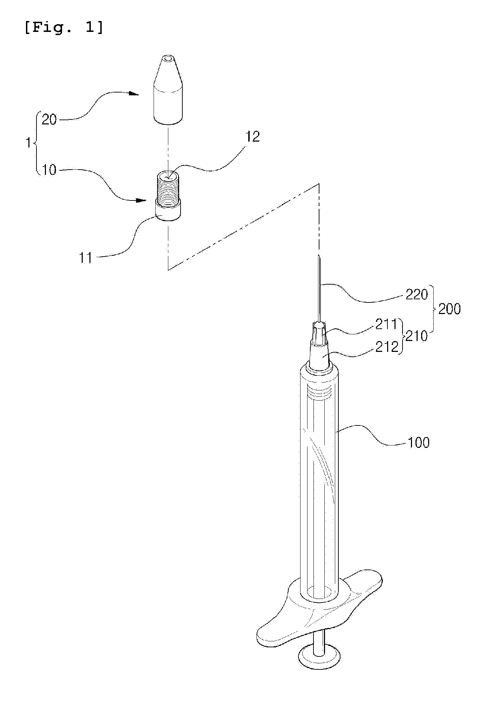

Fig. 1 is an exploded perspective view illustrating a tube assembly for controlling the length of a syringe needle according to an embodiment of the present invention.

The tube assembly 1 for controlling the length of a syringe needle according to an embodiment of the present invention includes a coupling body 10 coupled to a needle assembly 200 having a fixing member 210 coupled to an upper end of a syringe cylinder 100 and a syringe needle 220 coupled to the fixing member 210, the coupling body 10 including a coupling part 11 formed on a lower side thereof to be inserted into and coupled to the fixing member 210, and a through-hole 12 formed therein in order for the syringe needle 220 to penetrate the through-hole 12 and to protrude out of the front end; and a control tube 20 movably coupled to the coupling body 10 along a longitudinal direction of the syringe needle 220 to be able to control an exposure length of the syringe needle 220.

Medication using a syringe is to directly transfer an injection to the inside of the patient's body, and careful attention must be paid when the syringe is used. Diverse matters, such as danger of infection due to external foreign substances, patient's psychological burden, appropriate injection point and method, must be considered during injection.

Recently, diverse types of syringes that are individualized to match the purpose and function have been introduced. A typical syringe includes a cylinder 100 formed to store an injection, and a piston that applies pressure so that the injection inside the cylinder 100 is injected to the outside, and the needle assembly 200 is coupled to the front end portion of the cylinder 100. Fig. 1 illustrates the coupling state of the needle assembly 200 and the cylinder 100, and currently, this coupling type of the needle assembly 200 and the cylinder 100 has been widely used.

As described above, the present invention is to appropriately control the exposure length of the syringe needle 220 that is used in the existing syringe. The present invention can be used in all typical syringes provided with the syringe needles 220. Particularly, as illustrated in Fig. 1, it is appropriate that the present invention is used to be coupled to the needle assembly 200 which includes the fixing member 210 for fixing eh syringe needle 220.

The coupling body 10 is in the form of a cylinder as a whole. The coupling part 11 is formed on the lower side of the coupling body 10 to be coupled to the fixing member 210, and the through-hole 12 is formed in the coupling body 10 along the length direction. The coupling part 11 is fitted and fixed to one side of the fixing member 210, and the syringe needle 220 is exposed to the outside through the through-hole 12.

The coupling body 10 is coupled and fixed to the outer side of the fixing member 210, and the coupling body 10 is coupled to surround parts of the fixing member 210 and the syringe needle 220. The coupling body 10 may be formed with diverse lengths in consideration of the exposure length of the syringe needle 220.

The control tube 20 is movably coupled to the coupling part 11. It is preferable that the control tube 20 is in the form of a cylinder in consideration of the easiness of manufacturing and convenience in use. That is, the side surface of the control tube 20 is coupled to the coupling body 10, and the syringe needle 220 is exposed to the outside through the front end portion of the control tube 20. The control tube 20 may slide on the coupling body 10 in the length direction, or may be screw-engaged with the coupling body 10.

The front end portion of the control tube 20 is tapered so that the diameter thereof becomes reduced toward the front portion of the control tube 20. By forming the control tube as described above, the gap between the syringe needle 220 and the control tube 20 can be reduced, and the control tube 20 may be somewhat guided by the syringe needle 220 during its movement on the coupling body 10 to secure the stability of the control tube 20 and the syringe needle 220.

Since the lengths of the coupling body 10 and the control tube 20 determine the range of the exposure length of the syringe needle 220, the coupling body 10 and the control tube 20 can be formed with diverse lengths according to the purpose of the used syringe.

In the tube assembly for controlling the length of a syringe needle according to a preferred embodiment of the present invention, the coupling body 10 and the control tube 20 may be screw-engaged with each other.

In movably forming the control tube 20 on the coupling body 10, the control tube 20 may be formed to slide on the coupling body 10. The outer circumference of the coupling body 10 and the inner circumference of the control tube 20 are formed to be in contact with each other, and a constant friction force is generated between the coupling body 10 and the control tube 20 to control the position of the control tube 20.

However, in order to facilitate the position control of the control tube 20 and to prevent the control tube 20 from being pushed backward during injection, it is preferable that the coupling body 10 and the control tube 20 are screw-engaged with each other.

The coupling body 10 and the control tube 20 are in the form of a cylinder as a while, and screw thread is formed on the contact surfaces thereof so that the coupling body 10 and the control tube 20 are engaged with each other to be rotated with each other. It is preferable that the coupling body 10 and the control tube 20 may be formed of a plastic material, and in order to prevent the control tube 20 from easily moving on the coupling body 10 by an unintended external force, the coupling body 10 and the control tube 20 are formed and engaged with each other so that they are not loose on each other.

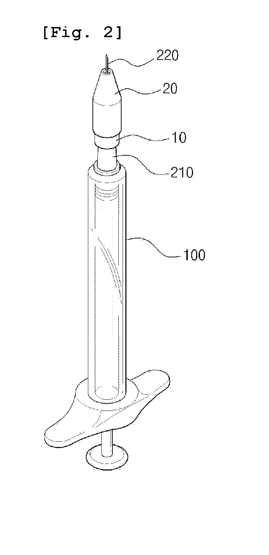

Fig. 2 is a perspective view illustrating a state where the tube assembly for controlling the length of a syringe needle illustrated in Fig. 1 is coupled to a needle assembly.

In the tube assembly for controlling the length of a syringe needle according to a preferred embodiment of the present invention, the control tube 20 is coupled to the outer side of the coupling body 10.

Screw thread is formed on the outer circumference of the coupling body 10, and screw thread is also formed on the inner circumference of the control tube 20 to be engaged with the screw thread of the coupling body 10.

The control tube 20 may be formed so that the outer surface thereof is movably coupled to the inner surface of the coupling body 10. However, in this case, it is not easy for a user to grip the control tube 20, and thus it may be difficult to perform position control of the control tube 20. According to the present invention, in consideration of this, the control tube 20 is formed to surround the coupling body 10. The user can grip the outer surface of the control tube 20 and can control the coupling body 10 to be put in a constant position against the coupling body 10.

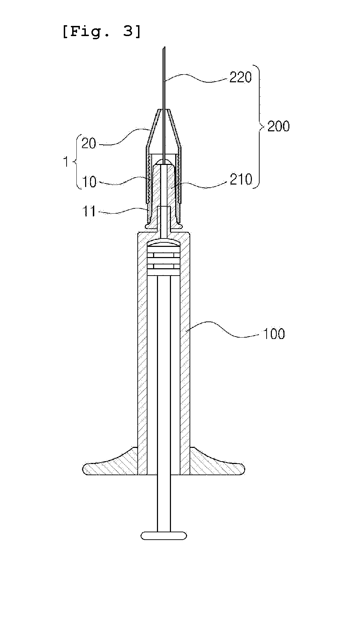

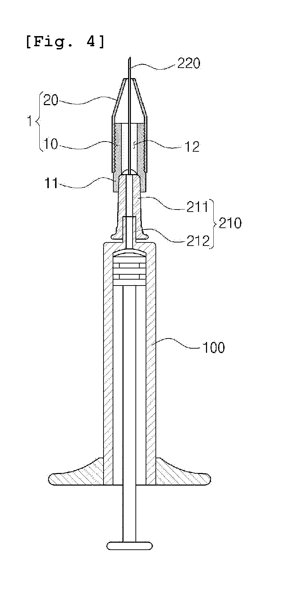

Figs. 3 and 4 are cross-sectional views illustrating a state where the tube assembly for controlling the length of a syringe needle according to another embodiment of the present invention is coupled to a fixing member of a needle assembly.

In the tube assembly 1 for controlling the length of a syringe needle according to another embodiment of the present invention, the coupling part 11 is coupled to the front end portion 211 of the fixing member 210, and an inner surface of the coupling part 11 is formed to correspond to the external shape of the fixing member 210, so that the coupling body 10 and the fixing member 210 are coupled to fit each other with no gap.

In the tube assembly 1 for controlling the length of a syringe needle according to the present invention, the coupling body 10 may be coupled to surround a fair part of the fixing member 210 as illustrated in Fig. 3.

In general, the needle assembly 200 is standardized, and has a constant shape. In a typical needle assembly 200, the fixing member 210 is formed so that the diameter of the rear end portion 212 thereof is somewhat larger than the diameter of the front end portion 211 thereof, and thus the shape of the needle assembly 200 is tapered as a whole. Thus, the front end portion 211 does not simply have a circular cross-sectional shape, but has a plurality of ribs to be substantially in "+" shape. Further, it is general that the rear end portion 212 of the fixing member 210 has a projection portion formed thereon. In the needle assembly 200 having the above-described shape, the coupling part 11 is formed so that the inner diameter of the lower end of the coupling part 11 is somewhat smaller than the outer diameter of the rear end portion 212 of the fixing member 210, and thus the coupling body 10 is compulsorily fitted to the fixing member 210 in a state where the coupling body 10 surrounds a fair part of the fixing member 210.

Unlike this, the coupling body 10 may be formed to be coupled to the fixing member 210 in a state where the coupling body 10 is positioned at the front end portion 211 of the fixing member 210. As described above, the cross-section of the front end portion 211 is substantially in "+" shape, and the inner surface of the coupling part 11 is shaped to correspond to this. In this case, the contact area between the fixing member 210 and the coupling body 10 is increased to achieve firmer coupling between them, and thus the coupling body 10 can be manufactured with a smaller diameter. Accordingly, the tube assembly 1 for controlling the length of a syringe needle can be coupled to the needle assembly 200 more stably.

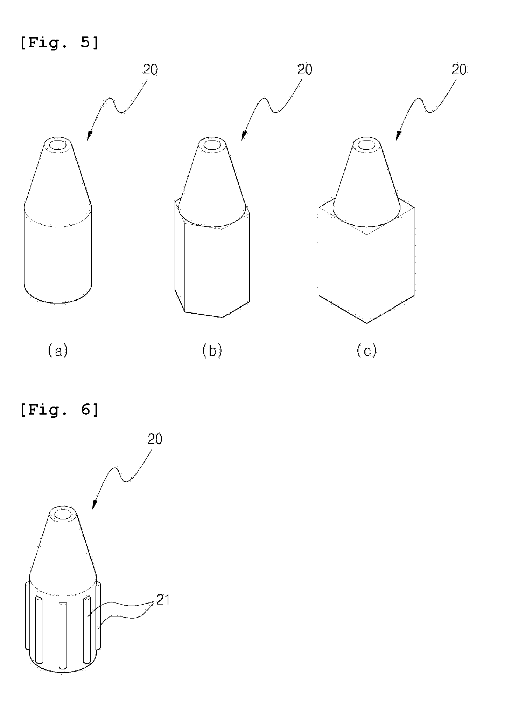

Fig. 5 is a perspective view and a cross-sectional view illustrating a control tube according to still another embodiment of the present invention.
In the tube assembly 1 for controlling the length of a syringe needle according to still another embodiment of the present invention, the control tube 20 has a circular or polygonal cross-sectional shape.

The outer circumference of the control tube 20 may be formed in a circular shape, but may have a polygonal cross-sectional shape. Since a user grips and moves the control tube 20 relatively to the coupling body 10, it may be difficult for the user to operate the control tube 20 in the case where the outer circumference thereof is simply in a circular shape. This is because since the control tube 20 is formed with a relatively small size, a portion of the control tube 20 that the user can grip is not large, and the user's hand may slip on the surface of the control tube 20. In consideration of this point, it is preferable that the outer circumference of the control tube 20 is formed so that the cross-section thereof is in a polygonal shape.

Unlike this, the outer circumference of the control tube 20 may be provided with a plurality of protrusions 21 formed thereon, or may be uneven. Fig. 6 illustrates the control tube 20 having protrusions 21 formed on the outer circumference of the control tube 20. This is also to prevent the sliding on the control tube 20 when the user grips the control tube 20.

### INDUSTRIAL APPLICABILITY

As apparent from the above description, according to the tube assembly for controlling the length of a syringe needle according to the present invention, since the exposure length of the syringe needle can be easily controlled using the coupling body that is coupled to the fixing member of the needle assembly and the control tube movably coupled to the coupling body, it is possible to control the exposure length of the syringe needle by simply mounting the tube assembly on the syringe having a typical syringe needle.

## Claims

1. A tube assembly for controlling the length of a syringe needle, comprising:
a coupling body coupled to a needle assembly having a fixing member coupled to an upper end of a syringe cylinder and a syringe needle coupled to the fixing member, the coupling body including a coupling part formed on a lower side thereof to be inserted into and coupled to the fixing member, and a through-hole formed therein in order for the syringe needle to penetrate the through-hole and to protrude out of the front end; and
a control tube movably coupled to the coupling body along a longitudinal direction of the syringe needle to be able to control an exposure length of the syringe needle.

2. The tube assembly according to claim 1, wherein the coupling body and the control tube are screw-engaged with each other.

3. The tube assembly according to claim 1, wherein the control tube is coupled to an outside of the coupling body.

4. The tube assembly according to claim 1, wherein the coupling part is coupled to a front end portion of the fixing member, and an inner surface of the coupling part is formed to correspond to an external shape of the fixing member, so that the coupling body and the fixing member are coupled to fit each other with no gap.

5. The tube assembly according to any one of claims 1 to 4, the control tube has a circular or polygonal cross-sectional shape.

6. The tube assembly according to any one of claims 1 to 4, wherein a plurality of protrusions are formed on an outer circumference of the control tube.
